# EUROPEAN PATENT SPECIFICATION

(11) **EP 3 216 860 B1**
(45) Date of publication and mention of the grant of the patent: **11.08.2021**
(21) Application number: 15857509.2
(22) Date of filing: 06.11.2015
(51) Int. Cl.: A61K 35/28, C12N 5/0775, A61P 19/02

(54) **LYSATES OF MESENCHYMAL STEM CELLS FOR THE TREATMENT OF SKELETAL MUSCLE INJURIES**
LYSATE VON MESENCHYMALEN STAMMZELLEN ZUR BEHANDLUNG VON SKELETTMUSKELVERLETZUNGEN
LYSATS DE CELLULES SOUCHES MÉSENCHYMATEUSES POUR LE TRAITEMENT DE TROUBLES MUSCULO-SQUELETTIQUES

(30) Priority: 06.11.2014 ES 201431630
(43) Date of publication of application: 13.09.2017
(73) Proprietor: Servicio Andaluz de Salud, 41092 Sevilla (ES); Universidad De Córdoba, 14004 Córdoba (ES)
(72) Inventor: MUÑOZ CASTAÑEDA, Juan Rafael, 14004 Córdoba (ES); DÍAZ TOCADOS, Juan Manuel, 14004 Córdoba (ES); DOMÍNGUEZ PÉREZ, Juan Manuel, 14004 Córdoba (ES)
(74) Representative: Hoffmann Eitle
(86) International application number: PCT/ES2015/070797
(87) International publication number: WO 2016/071555

(56) References cited:
- WO-A2-2009/134429
- US-A1- 2011 020 291
- US-A1- 2012 225 130
- BASHIR JAMIL ET AL: "Mesenchymal Stem Cell Therapies in the Treatment of Musculoskeletal Diseases", PM&R, ELSEVIER, AMSTERDAM, NL, vol. 6, no. 1, 15 January 2014 (2014-01-15), pages 61-69, XP028809118, ISSN: 1934-1482, DOI: 10.1016/J.PMRJ.2013.05.007
- ROTH VON P ET AL: "Immediate local transplantation of mesenchymal stem cells into a severely injured skeletal muscle in rats improves the functional outcome comparable to delayed transplantation", INFLAMMATION RESEARCH, BIRKHAEUSER VERLAG, BASEL, CH, vol. 59, no. 1, 1 March 2010 (2010-03-01), page S42, XP002653347, ISSN: 1023-3830
- TOBIAS WINKLER ET AL: "Dose?Response Relationship of Mesenchymal Stem Cell Transplantation and Functional Regeneration After Severe Skeletal Muscle Injury in Rats", TISSUE ENGINEERING PART A, MARY ANN LIEBERT, vol. 15, no. 3, 30 July 2008 (2008-07-30), pages 487-492, XP002653350, ISSN: 1937-3341, DOI: 10.1089/TEN.TEA.2007.0426 [retrieved on 2008-08-04]
- HENG B. C. ET AL.: 'An autologous cell lysate extract from human embryonic stem cell (hESC) derived osteoblasts can enhance osteogenesis of hESC' TISSUE & CELL vol. 40, no. 3, ISSN 0040-8166 pages 219 - 228, XP022609498
- TOH W. S. ET AL.: 'Advances in mesenchymal stem cell -based strategies for cartilage repair and regeneration.' STEM CELL REVIEWS vol. 10, no. 5, 29 May 2014, ISSN 1558-6804 pages 686 - 696, XP035392625
- None

## Description

### FIELD OF THE INVENTION

The present invention is comprised in the field of biology and medicine, and relates to the use of the intracellular content of human mesenchymal stem cells (MSCs) in the production of a medicinal product for cell, tissue and organ regeneration. Preferably, the present invention relates to the use of the intracellular content of human mesenchymal stem cells (MSCs) in the production of a medicinal product for the treatment of skeletal muscle injuries, and preferably for the treatment of osteoarthrosis.

### BACKGROUND OF THE INVENTION

The administration of mesenchymal stem cells into an organism presents a number of problems. When the administered cells originate from the same individual, i.e., autologous transplant, the main problems are related to cell handling and dose optimization. Furthermore, the cells may be hard to control and there is the possibility of creating other problems, for example, tumor generation (Koh and Kang, 2012. EMBO Rep., 13(5): 412-422).

On the other hand, when the administered cells originate from another individual, i.e., allogeneic transplant, problems associated with compatibility arise and during the receptor immunosuppression process. MSCs have immunomodulating properties so they are indicated for preventing and reducing rejection. However, the use of cells of this type or the therapy therewith has been linked to tumor formation (Mishra et al., 2008. Cancer Research 68 (11), 4331-4339).

Part of the effects triggered by MSCs is caused by the cell-cell interactions brought about by membrane proteins. MSCs can be lysed through cell sonication and the membrane protein-free intracellular content can be obtained after centrifugation. The exclusive use of the intracellular content of mesenchymal stem cells could help to prevent these problems.

It is known that degenerative joint disease or osteoarthritis is a chronic-type joint pathology with a high incidence in mammals. It is an articular cartilage degenerative process. In arthrosis, this process begins at the articular cartilage level, involving all the components of the joint during its development. It has a multifactorial etiology with the intervention of genetic factors, metabolic factors, physical factors, etc.

Osteoarthritis treatment varies according to the severity of the symptoms and focuses on reducing pain and improving joint movement. The treatments fundamentally consist of:
- Plans combining drugs, rest, physical activity, protection of joints, use of heat or cold to reduce pain and physical or occupational therapy.
- Weight loss
- Use of medicinal products such as acetaminophen or non-steroidal anti-inflammatory drugs (NSAIDs) to help to relief joint pain, stiffness and swelling.
- Physical activity to keep joints flexible and to maintain or improve muscle strength
- Protection of joints to prevent damage or strain in joints with pain.
- Corticosteroids or hyaluronic acid derivatives can be injected into joints that do not respond to other treatments.
- In certain studies relating to the knee, dietary supplements such as glucosamine and chondroitin have been shown to relief pain and improve articular function in severe osteoarthritis.
- Surgery for advanced osteoarthritis associated with articular damage and/or clear limitations in the function of the joints.

In animals, skeletal muscle injuries are treated with different types of medicinal products that aim to reduce inflammation and pain. However, when the degenerative process progresses, these drugs are neither capable of promoting mobility nor reducing pain. Therapies such as infusion of mesenchymal stem cells or treatment with platelet-derived growth factors are two of the alternatives that are currently being evaluated and marketed in some cases, although the efficacy thereof is still not completely proven. In any case, in order to perform these treatments today, these therapies require either a prior surgery on the animal for extracting the bone marrow or adipose tissue or a prior hospitalization for factor extraction and formation. With respect to treatment with factors, there is no consensus as regards the protocol for extracting and creating these platelet-derived factors, so disparity in the results is often justified.

Finally, it must be indicated that there is currently no sufficient evidence to indicate if stem cells exert a beneficial effect due to differentiation into the cell type of the damaged organ or, in contrast, if the effect is mediated by cytokines and factors acting on resident cells in a paracrine manner. The positive effect found with the application of lysates of MSCs reveals that paracrine actions are extremely powerful and effective. In this sense, the invention by delivering this concentrate into the damaged area, accelerates regeneration as it would increase the bioavailability of the molecules required for regenerating the tissue.

### BRIEF DESCRIPTION OF THE INVENTION

A **first aspect** of the invention relates to an intracellular content of at least one isolated mesenchymal stem cell for use in the prevention, relief or treatment of osteoarthrosis, wherein the isolated mesenchymal stem cell is obtained from a different individual from which the intracellular content will be administered and wherein said intracellular content of at least one isolated mesenchymal stem cell is infused in the site of injury.

In another preferred embodiment of this aspect of the invention, the isolated mesenchymal stem cell is a cell of a mammal. In another preferred embodiment, it is a mesenchymal cell of a dog. In another preferred embodiment, it is a mesenchymal cell of a horse. In another preferred embodiment, it is a mesenchymal cell of a human.

A **second aspect** of the invention relates to a composition comprising an intracellular content of at least one isolated mesenchymal stem cell for use in the prevention, relief or treatment of osteoarthrosis, wherein the isolated mesenchymal stem cell is obtained from a different individual from which the intracellular content will be administered and wherein said composition is infused in the site of injury.

In another preferred embodiment of this aspect of the invention, the isolated mesenchymal stem cell is a cell of a mammal. In another preferred embodiment of this aspect of the invention, the isolated mesenchymal stem cell is a cell of a dog. In another preferred embodiment of this aspect of the invention, the isolated mesenchymal stem cell is a mesenchymal cell of a horse. In another preferred embodiment of this aspect of the invention, the isolated mesenchymal stem cell is a cell of a human.

### BRIEF DESCRIPTION OF THE DRAWINGS

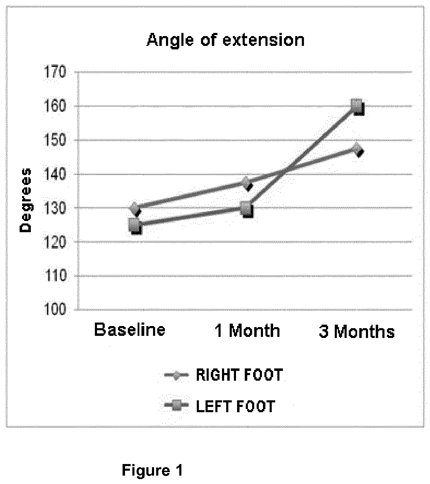

**Figure 1****.** Assessment of the angle of extension. Hip joint extension is the most painful and restrictive joint movement in hip osteoarthrosis in dogs. The assessment of the angle of extension records an increase in the value thereof after 1 month with respect to the baseline value, this increase in the datum become more evident after 3 months, which indicates a significant improvement in the range of joint mobility after therapy.

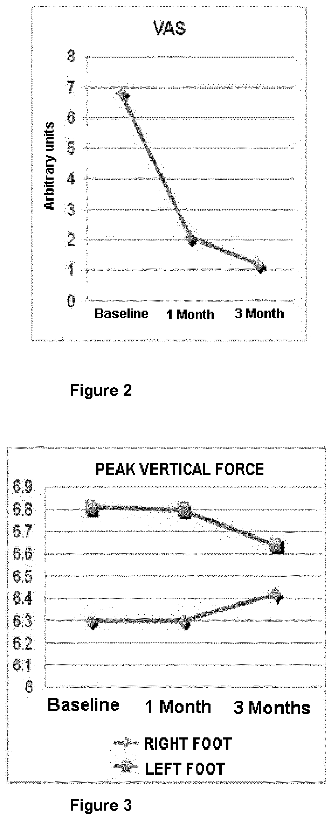

**Figure 2****.** VAS (Subjective pain assessment). The average assessment of the degree of pain the owner assigns to their animal shows a significant decrease after 1 month, dropping further after 3 months, which indicates that the treatment reduces the pain the owner thinks their animal feels.
**Figure 3****.** Peak vertical force. In osteoarthrosis affecting both hips, the patients experience bilateral lameness, and one joint has a more advanced degree of OA. This means that in the baseline condition, the patient bears less weight (lower PVF) on the limb the hip of which experiences greater degeneration, and places more load to bear its weight on the limb the hip of which is better (greater PVF). The treatment causes the pain to disappear from both joints, the patient therefore distributes the bearing force between both pelvic limbs, the effect is that for the hip which was better at the baseline level and shown greater PVF, this value decreases after treatment, whereas for the hip with lower PVF, this bearing force value increases. This equilibrium in the PVF parameter for both pelvic limbs indicates improvement as a result of the therapy.

### DETAILED DESCRIPTION OF THE INVENTION

The authors of the present invention have evaluated the beneficial effect of the intracellular content of mesenchymal cells (MSCs) in skeletal muscle injuries. Furthermore, they have found a series of differences indicating that the infusion of intracellular content of mesenchymal stem cells has advantages over the transplant of these cells intact.

A **first aspect** of the invention relates to an intracellular content of at least one isolated mesenchymal stem cell for use in the prevention, relief or treatment of osteoarthrosis, wherein the isolated mesenchymal stem cell is obtained from a different individual from which the intracellular content will be administered and wherein said intracellular content of at least one isolated mesenchymal stem cell is infused in the site of injury.

The mesenchymal stem cells are obtained from a tissue or an organ of an individual in a growth stage after the embryonic stage, preferably from the bone marrow thereof. Preferably, the stem cells of the investigation were isolated in a post-natal stage. Preferably, they were isolated from a mammal, and more preferably from a human, including newborns, children, adolescents and adults.

In a preferred embodiment of this aspect of the invention, the isolated mesenchymal stem cell is a cell of a mammal. In another preferred embodiment, it is a mesenchymal cell of a dog. In another preferred embodiment, it is a mesenchymal cell of a horse. In another preferred embodiment, it is a mesenchymal cell of a human.

The term "individual" includes any animal, particularly, vertebrate animals, preferably mammals, such as mice, rats, horses, pigs, rabbits, cats, sheep, dogs, cows, human beings, etc. As it is understood in the specification, the term mammal refers to any organism of the *Eukaryota* superkingdom, *Metazoa* kingdom, *Chordata* phylum, *Mammalia* class. The blood can therefore be obtained from the coronary sinus of a mouse, rat, pig, dog, horse and human. In another preferred embodiment, the mammal is a human being. In another preferred embodiment, the mammal is a dog. In another preferred embodiment, the mammal is a horse.

If desired, the mesenchymal stem cells that give rise to the intracellular lysate for use according to the invention can be genetically modified by any conventional method including, in an illustrative and non-limiting manner, transgenesis processes, deletions or insertions in the genome of said stem cells modifying the expression of genes that are important for their basic properties (proliferation, migration, differentiation, etc.), or by means of the insertion of nucleotide sequences encoding proteins of interest such as, for example, proteins with therapeutic properties. Therefore, in another preferred embodiment the cell of the disclosure was genetically modified.

The progeny of a single clone cell can be expanded by means of several passes without suffering from any apparent chromosomal abnormality or the loss of growth and differentiation properties.

Therefore, if desired, the mesenchymal cells can be expanded clonally using a suitable method for cloning cell populations. For example, a proliferated population of cells can be physically collected and seeded in a different plate (or the wells of a multi-well plate). Another option is that the cells can be subcloned in a multi-well plate at a statistical ratio to make the operation of placing a single cell in each well easier (for example, from about 0.1 to about one cell/well or even from 0.25 to 0.5 cells/well, such as 0.5 cells/well, for example). Naturally, the cells can be cloned at a low density (for example, in a Petri dish or another suitable substrate) and isolated from other cells using devices such as cloning rings. The production of a clone population can be expanded in any suitable culture medium. In any case, the isolated cells can be cultured to a suitable point where their development phenotype can be evaluated.

The term "isolated" indicates that the cell or the cell population of the disclosure to which reference is made are not found in its natural environment. In other words, the cell or the cell population was separated from its surrounding tissue. Particularly, it means that said cell or the cell population is substantially devoid (free) of other cells normally present in the tissue from which they were isolated, i.e., when it is separated from at least 60%, preferably at least 80%, preferably at least 90%, more preferably at least 95%, even more preferably at least 96%, 97%, 98% or even 99%, of other cells present in the tissue from which they were isolated.

It also refers to cells or cell populations that were isolated from the organism from which they originate. The term also includes cells that were isolated from one organism and re-introduced into the same or in another organism.

The term "stem cell" refers to a cell with the capacity to clone itself, renew itself and differentiate into multiple cell lineages. Particularly, mesenchymal stem cells have the capacity to proliferate extensively and form fibroblastic cell colonies. As it is used herein, the expression "stem cell" refers to a totipotent, pluripotent or multipotent cell which is capable of generating one or more types of differentiated cells, and furthermore has the capacity to regenerate itself, i.e., to produce more stem cells. The "totipotent stem cells" can give rise both to embryonic components (such as, the three embryonic layers, the germline lineage and tissues that will give rise to the yolk sac, for example), and to extraembryonic components (such as placenta). In other words, they can form all the cell types and give rise to a whole organism. "pluripotent stem cells" can form any type of cell corresponding to the three embryonic lineages (endoderm, ectoderm and mesoderm), as well as the germline and yolk sac. They can therefore form cell lineages but a whole organism cannot be formed from them. "Multipotent stem cells" are those that can only generate cells from the same layer or embryonic lineage of origin. The bone marrow stores at least two different stem cell populations: mesenchymal stem cells (MSCs) and hematopoietic stem cells (HSCs). In the context of the present disclosure, the stem cells are selected from the group comprising mesenchymal stem cells, hematopoietic stem cells, embryonic stem cells, induced pluripotent stem cells, adult stem cells, or combinations thereof. In a particular aspect, the stem cells are stem cells of a mammal, preferably humans. In a particular aspect, the stem cells are mesenchymal stem cells, preferably human mesenchymal stem cells.

The term "adult stem cell" refers to that a stem cell that is isolated from a tissue or an organ of an animal in a growth stage after the embryonic stage. Preferably, the stem cells of the invention are isolated in a post-natal stage. Preferably, they are isolated from a mammal, and more preferably from a human, including newborns, children, adolescents and adults. Adult stem cells can be isolated from a large variety of tissues and organs, such as bone marrow (mesenchymal stem cells, multipotent adult progenitor cells and hematopoietic stem cells), adipose tissue, cartilage, epidermis, hair follicle, skeletal muscle, heart muscle, intestine, liver, neurons.

As it is used herein, the term "mesenchymal stem cell" or "MSC" refers to a multipotent stromal cell originated from the mesodermal germ layer which can differentiate into different types of cells, including osteocytes (bone cells), chondrocytes (cartilage cells) and adipocytes (fat cells). The markers expressed by the mesenchymal stem cells include CD105 (SH2), CD73 (SH3/4), CD44, CD90 (Thy-1), CD71 and Stro-1 as well as adhesion molecules CD106, CD166, and CD29. The negative markers for MSCs (not expressed) include, among others, hematopoietic markers CD45, CD34, CD14, and costimulatory molecules CD80, CD86 and CD40 as well as the adhesion molecule CD31. The MSCs can be obtained, without limitation, from bone marrow, adipose tissue (such as subcutaneous adipose tissue), liver, spleen, testicles, menstrual blood, amniotic fluid, pancreas, periosteum, sinovial membrane, skeletal muscle, dermis, pericytes, trabecular bone, human umbilical cord, lung, dental pulp and peripheral blood. The MSCs according to the invention can be obtained from any of the preceding tissues, such as from bone marrow, subcutaneous adipose tissue or umbilical cord. The MSCs can be isolated from bone marrow by means of methods known by the person skilled in the art. Generally, said methods consist of isolating mononuclear cells by means of density gradient centrifugation (Ficoll, Percoll) of bone marrow aspirates, and then seeding the isolated cells in tissue culture plates in medium containing fetal bovine serum. These methods are based on the capacity of the MSCs to adhere to plastic, such that while non-adhered cells are removed from the culture, adhered MSCs can be expanded in culture plates. The MSCs can also be isolated from subcutaneous adipose tissue following a similar method known by the person skilled in the art. A method for isolating MSCs from bone marrow or subcutaneous adipose tissue has been described previously (de la Fuente et al., Exp. Cell Res. 2004, Vol. 297: 313:328). In a particular embodiment of the invention, the mesenchymal stem cells are obtained from umbilical cord, preferably from human umbilical cord.

The mesenchymal stem cells according to the present disclosure can be obtained by means of methods and methodologies known by the person skilled in the art from a tissue of a subject. First, the sample comprising the stem cells is preferably washed in order to separate the fraction comprising the mesenchymal stem cells from the other material. In a particular embodiment, the tissue sample is washed with a physiologically compatible saline solution, such as a phosphate buffered saline or PBS.

Methods of purifying stem cells after obtaining same have been widely described in the art and include, without limitation, density gradient centrifugation (by means of Ficoll-Hypaque, for example) followed by incubation of adherent cells, cell cytometry with sorting and incubation with marker-specific magnetic particles (with positive or negative selection).

As understood by the person skilled in the art, the total number of stem cells that can be obtained from an individual can be larger or smaller depending on the tissue from which said stem cells originate. Therefore, particularly, the total number of mesenchymal stem cells that can be obtained from adipose tissue is larger than the total number that can be obtained from bone marrow, for example.

The term "osteoarthritis", also known as osteoarthrosis, arthrosis or degenerative joint disease, is the most common type of arthritis. Osteoarthritis is a chronic condition characterized by wear of the joint cartilage. Wear of the cartilage causes the bones to rub against one another, resulting in stiffness, pain and loss of joint movement. Osteoarthritis typically affects certain joints, such as hips, hands, knees, lumbar area (lower back) and neck. Above 50 years of age, women suffer from osteoarthritis more often than men do. The symptoms typically start from 40 years old and progress slowly. The main symptoms of osteoarthritis are: joint pain or stiffness after periods of inactivity or excessive use, grating or gripping sensation during joint movement and bone growths at the edges of the affected joints. The possible causes of osteoarthritis are unknown although there are, however, certain factors which increase the risk of developing the disease: heredity, overweight, joint injuries, excessive or repetitive use of some joints, lack of physical activity, nerve damage and aging.

### PHARMACEUTICAL COMPOSITION OF THE DISCLOSURE

Another aspect of the invention relates to a composition comprising an intracellular content of at least one isolated mesenchymal stem cell for use in the prevention, relief or treatment of osteoarthrosis, wherein the isolated mesenchymal stem cell is obtained from a different individual from which the intracellular content will be administered and wherein said composition is infused in the site of injury, hereinafter composition of the invention. Preferably, the isolated mesenchymal stem cell is a human cell. More preferably, the composition is a pharmaceutical composition. Even more preferably, the composition further comprises pharmaceutically acceptable excipients.

The intracellular content of the composition for use according to the invention is obtained from mesenchymal cells comprising an isolated cell or an isolated population of the disclosure. Said composition is obtained from mesenchymal stem cells and can contain a medium in which the cells are found; said medium must be compatible with said cells, for example, but without limitation, isotonic solutions, optionally supplemented with serum; cell culture medium or, alternatively, a solid, semisolid, gelatinous or viscous support medium.

The composition for use according to the invention can also contain the secretome of mesenchymal stem cells. In this invention, secretome is understood as the totality of proteins secreted into the extracellular space by a given cell, tissue or organ. Normally, the secreted proteins are involved in various physiological processes such as cell signaling.

The composition for use according to the invention can in turn form part of a pharmaceutical composition for the administration thereof to a subject. Therefore, another aspect of the invention relates to a pharmaceutical composition, comprising an intracellular content of at least one isolated mesenchymal stem cell for use in the prevention, relief or treatment of osteoarthrosis, wherein the isolated mesenchymal stem cell is obtained from a different individual from which the intracellular content will be administered and wherein said composition is infused in the site of injury. In a preferred embodiment, the pharmaceutical composition for use according to the invention further comprises a pharmaceutically acceptable vehicle. In another preferred embodiment, the pharmaceutical composition for use according to the invention further comprises another active ingredient. The term "pharmaceutically acceptable vehicle" refers to a vehicle that must be approved by a regulatory agency of a federal government or state government or listed in the United States Pharmacopeia or the European Pharmacopeia, or another generally recognized pharmacopeia for the use thereof in animals, and more specifically in humans.

The term "vehicle" refers to a diluent, co-adjuvant, excipient or carrier with which the intracellular content of the mesenchymal cells of the invention or of said composition comprising the intracellular content of mesenchymal stem cells obtainable according to the method of the invention must be administered; obviously, said vehicle must be compatible with said intracellular content.

If desired, the pharmaceutical composition of the disclosure can also contain, when necessary, additives for increasing, controlling or otherwise regulating the desired therapeutic effect of the intracellular content of the mesenchymal stem cells, which comprise said pharmaceutical composition, and/or auxiliary substances or pharmaceutically acceptable substances, such as buffering agents, surfactants, cosolvents, preservatives, etc. Said pharmaceutically acceptable substances which can be used in the pharmaceutical composition of the invention are generally known by the persons skilled in the art and are normally used in the production of cell compositions. Examples of suitable pharmaceutical vehicles are described, for example, in "Remington's Pharmaceutical Sciences", of E.W. Martin. Additional information about said vehicles can be found in any manual of pharmaceutical technology (Galenical Pharmacy).

As it is used herein, the term "active ingredient", "active substance", "pharmaceutically active substance", or "pharmaceutically active ingredient" means any component that may provide a pharmacological activity or another different effect in the diagnosis, cure, mitigation, treatment, or prevention of a disease, or that affects the body structure or function of humans or other animals. The term includes those components that promote a chemical change in the production of the drug and are present in the drug in an envisaged modified form, providing the specific activity or effect.

In another preferred aspect of this aspect of the invention, the isolated mesenchymal stem cell present in the composition or pharmaceutical composition of the disclosure, is a cell of a mammal. In another preferred aspect of the disclosure, the isolated mesenchymal stem cell is a cell of a dog. In another preferred aspect of the disclosure, the isolated mesenchymal stem cell is a mesenchymal cell of a horse. In another preferred aspect of the disclosure, the isolated mesenchymal stem cell is a cell of a human. As it is used herein, the term "medicinal product" refers to any substance used for the prevention, diagnosis, relief, treatment or cure of diseases in humans and animals.

The pharmaceutical composition of the disclosure will contain a prophylactically or therapeutically effective amount of the intracellular content of mesenchymal stem cells to provide the desired therapeutic effect. As it is used herein, the term "prophylactically or therapeutically effective amount" refers to the amount of the intracellular content of mesenchymal stem cells contained in the pharmaceutical composition which is capable of producing the desired therapeutic effect and, will generally be determined, among other factors, by the actual characteristics of the intracellular lysate of the cells and the desired therapeutic effect to be achieved. Generally, the therapeutically effective amount of the intracellular content of the disclosure that must be administered will depend, among other factors, on the actual characteristics of the subject, the severity of the disease, the dosage form, etc. For this reason, the doses mentioned in this invention must be taken into account only as guidance for the person skilled in the art who must adjust this dose depending on the factors described above.

The pharmaceutical composition of the disclosure will be formulated according to the chosen dosage form. The pharmaceutical composition of the disclosure can be prepared in a liquid or gel dosage form, for example, in the form of a suspension, to be injected or perfused to the individual.

The pharmaceutical composition of the disclosure will be administered to the individual by conventional means. For example, said pharmaceutical composition can be administered to said individual using suitable devices, such as syringes, catheters (a standard peripheral intravenous catheter, a central venous catheter or a pulmonary artery catheter, etc.), trocars, cannulas, etc. In all cases, the pharmaceutical composition of the invention will be administered using the equipment, apparatus and devices suitable for the administration of cell compositions and known by the person skilled in the art. As understood by the person skilled in the art, the direct administration of the pharmaceutical composition of the disclosure to the site meant to benefit from said administration can sometimes be advantageous. therefore, the direct administration of the pharmaceutical composition of the disclosure to the desired organ or tissue can be achieved by direct administration (by injection, etc.) on the outer surface of the affected organ or tissue by means of inserting a suitable device, e.g., a suitable cannula, by arterial or venous perfusion (including backflow mechanisms) or by other means mentioned herein or known in the art.

If desired, the pharmaceutical composition of the disclosure can be stored up to the moment of application thereof by means of the conventional methods known by the persons skilled in the art. This pharmaceutical composition can also be stored together with additional medicinal products, useful in the treatment of diseases, in an active form comprising a combined therapy. As mentioned above, if desired, the mesenchymal stem cells that give rise to the intracellular content of the disclosure can be genetically modified by any conventional method including, in an illustrative and non-limiting manner, transgenesis processes, deletions or insertions in the genome of said stem cells modifying the expression of genes that are important for their basic properties (proliferation, migration, transdifferentiation, etc.).

In another preferred aspect, the composition of the disclosure further comprises a pharmaceutically acceptable vehicle. In another preferred aspect, the composition of the disclosure further comprises another active ingredient or therapeutic agent. Said therapeutic agent is preferably selected from an analgesic agent (in the treatment of inflammation and pain) or an anti-infective agent (in the prevention of infection).

In particular, non-limiting examples therapeutic agents which are useful according to the invention include the following therapeutic categories: analgesics, such as non-steroidal anti-inflammatory drugs, opiate agonists and salicylates; anti-infective agents, such as anthelmintics, antianaerobics, antibiotics, aminoglycoside antibiotics, antifungal antibiotics, cephalosporin, macrolide antibiotics, miscellaneous beta-lactam antibiotics, penicillin, quinolone antibiotics, sulfonamide antibiotics, tetracycline antibiotics, antimycobacterials, antituberculosis antimycobacterials, antiprotozoals, antimalarial antiprotozoals, antiviral agents, antiretroviral agents, scabicides, anti-inflammatory agents, anti-inflammatory corticosteroids, topical local anesthetics/antipruritics, anti-infectives, anti-infective topical antimycotics, anti-infective topical antiviral agents, electrolytic and renal agents, such as acidifying agents, alkalinizing agents, diuretics, carbonic anhydrase inhibitors, diuretics, loop diuretics, osmotic diuretics, potassium-sparing diuretics, thiazide diuretics, electrolyte replacements, and uricosuric agents; enzymes, such as pancreatic enzymes and thrombolytic enzymes; gastrointestinal agents, such as antidiarrheals, antiemetics, gastrointestinal anti-inflammatory agents, salicylate of anti-inflammatory agents, antacid anti-ulcer agents, acid-pump inhibitor anti-ulcer agents, gastric mucosal H2 blocker anti-ulcer agents, anti-ulcer agents, cholelitholytic agents, digestants, emetics, laxatives and stool softeners, and prokinetic agents, general anesthetics such as inhaled halogenated anesthetics, inhaled anesthetics, intravenous anesthetics, barbiturates, benzodiazepines, intravenous anesthetics, opiate intravenous anesthetics and intravenous agonist anesthetics, hormones and hormone modifiers, such as abortifacients, corticosteroid adrenal agents, adrenal agents, androgens, anti-androgens, immunobiological agents, such as immunoglobulins, inmunosuppressants, toxoids, and vaccines; local anesthetics, such as amide of local anesthetics and ester-type local anesthetics, musculoskeletal agents, such as anti-gout anti-inflammatory agents, corticosteroid anti-inflammatory agents, gold compound anti-inflammatory agents, immuno-suppressive anti-inflammatory agents, non-steroidal anti-inflammatory drugs (NSAIDs), salicylate anti-inflammatory agents, minerals and vitamins, such as vitamin A, vitamin B, vitamin C, vitamin D, vitamin E and vitamin K.

In a particular embodiment, the therapeutic agents which are useful according to the preceding categories include: (1) analgesics, in general, such as lidocaine or the derivatives thereof, and analgesic non-steroidal anti-inflammatory drugs (NSAIDs), including diclofenac, ibuprofen, ketoprofen, and naproxen; (2) analgesic opiate agonists, such as codeine, fentanyl, hydromorphone and morphine; (3) salicylate analgesics, such as aspirin (ASA); (4) antihistamine HI-blocker, such as terfenadine, clemastine and (5) anti-infective agents, such as mupirocin; (6) antianaerobic anti-infectives, such as chloramphenicol and clindamycin; (7) antifungal antibiotic anti-infectives, such as amphotericin b, clotrimazole, fluconazole, and ketoconazole; (8) anti-infective macrolide antibiotics, such as azithromycin and erythromycin; (9) miscellaneous anti-infective beta-lactam antibiotics, such as aztreonam and imipenem; (10) anti-infective penicillin antibiotics, such as nafcillin, oxacillin, penicillin G, and penicillin V and; (11) anti-infective quinolone antibiotics, such as ciprofloxacin and norfloxacin; (12) anti-infective tetracycline antibiotics, such as doxycycline, minocycline, and tetracycline; (13) anti-infective antituberculosis antimycobacterials such as isoniazid (INH), and rifampin and; (14) anti-infective antiprotozoals, such as atovaquone and dapsone; (15) anti-infective antimalarial antiprotozoals, such as chloroquine and pyrimethamine; (16) anti-infective anti-retrovirals, such as ritonavir and zidovudine; (17) antiviral anti-infective agents, such as acyclovir, ganciclovir, interferon alpha, and rimantadine;; (18) topical anti-infective antifungals, such as amphotericin B, clotrimazole, miconazole, and nystatin and; (19) topical anti-infective antivirals, such as acyclovir; (20) electrolytic and renal agents, such as lactulose; (21) loop diuretics, such as furosemide; (22) potassium-sparing diuretics, such as triamterene; (23) thiazide diuretics, such as hydrochlorothiazide (HCTZ); (24) uricosuric agents, such as probenecid; (25) enzymes such as RNase and DNase; (26) antiemetics, such as prochlorperazine; (27) salicylate gastrointestinal anti-inflammatory agents, such as sulfasalazine; (28) acid-pump inhibitor anti-ulcer agents, such as omeprazole; (29) H2 blocker anti-ulcer agents, such as cimetidine, famotidine, nizatidine, and ranitidine; (30) digestants, such as pancrelipase; (31) prokinetic agents, such as erythromycin; (32; ester) local anesthetics, such as benzocaine and procaine; (33) musculoskeletal corticosteroid anti-inflammatory agents, such as beclomethasone, betamethasone, cortisone, dexamethasone, hydrocortisone, and prednisone; (34) musculoskeletal anti-inflammatory immunosuppressants, such as azathioprine, cyclophosphamide, and methotrexate; (35) musculoskeletal non-steroidal anti-inflammatory drugs (NSAIDs), such as diclofenac, ibuprofen, ketoprofen, ketorlac, and naproxen; (36) minerals, such as iron, calcium and magnesium; (37) the vitamin B compounds, such as cyanocobalamin (vitamin B12) and niacin (vitamin B3); (38) vitamin C compounds, such as ascorbic acid; and (39) vitamin D compounds, such as calcitriol.

In another preferred embodiment, the therapeutic agent can be a growth factor or another molecule affecting cell differentiation and/or proliferation, such as for example, but without limitation, platelet-derived growth factor (PDGF), transforming growth factor (TGF), insulin-like growth factor (IGF), hepatocyte growth factors (HGF), epidermal growth factor (EGF), vascular endothelial growth factors (VEGF), fibroblast growth factor (FGF), or any of the combinations thereof. The growth factors inducing final differentiation states are well known in the art and can be selected from any of those factors that has proven to induce a final differentiation state. The growth factors for use in the methods described herein can be, in certain embodiments, variants or fragments of a naturally occurring growth factor. For example, a variant can be generated by making conservative substitutions of amino acids and testing the resulting variant in one of the functional assays described above or another functional assay known in the art. Conservative substitution of amino acids refers to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide in the side chains is asparagine and glutamine; a group of amino acids having side aromatic chains is phenylalanine, tyrosine and tryptophan; a group of amino acids having side basic chains is lysine , arginine and histidine, and a group of amino acids having sulfur in the side chains is cysteine and methionine. Preferred groups for amino acid substitution are: valine-leucine-isoleucine, phenylalanine-tyrosine, lysine-arginine, alanine-valine, and asparagine-glutamine.

As will be seen by persons skilled in the art, the variants or fragments of the polypeptide growth factors can be generated using conventional techniques, such as mutagenesis, including the creation of discrete point mutation (s), or by truncation. For example, the mutation can give rise to variants substantially conserving the same, or simply a subset of the, biological activity of a polypeptide growth factor from which it was derived.

In another preferred embodiment, the therapeutic agent can be a Plasma Rich in Growth factors PRGF. It is based on the use of platelets as vehicle for the controlled release of different cell signals which accelerate and optimize the repair of tissues damaged due to a number of reasons such as a surgical treatment, injury or disease.

Throughout the description and claims the word "comprises" and variants thereof do not seek to exclude other technical features, additions, components or steps. For those skilled in the art, other objects, advantages and features of the invention will be inferred in part from the description and in part from putting the invention into practice. The following examples and drawings are provided by way of illustration and do not seek to limit the present invention.

### EXAMPLES OF THE INVENTION

The following example is provided by way of illustration and do not seek to limit the present invention.

### MATERIAL AND METHODS

All the animal care and experimental methods were approved by the Ethics and Research Committee of Hospital Universitario Reina Sofía/IMIBIC according to Directive 2010/63/EU of the European Parliament and institutional journals for the use and care of laboratory animals.

### Isolation of mesenchymal stem cells (MSCs) from the adipose tissue of a dog

The MSCs were extracted from the adipose tissue adjacent to the falciform ligament of donor female dogs which has been subjected to an ovariectomy operation. The tissue was disinfected with 100 mM of phosphate-buffered saline (PBS) pH=7.4 (Sigma-Aldrich, St. Louis MO) containing penicillin and streptomycin in a preventive manner under a sterile condition. Repeated washing with PBS was then performed, removing the blood vessels present. The tissue was ground and the same volume of a digestion solution was added based on the alpha minimum essential culture medium (α-MEM) (Sigma-Aldrich, St. Louis MO) supplemented with 1 mg/ml of collagenase (Sigma-Aldrich, St. Louis MO). The tissue with the digestion medium was kept at 37°C for 1 hour. After the digestion time has lapsed, the collagenase was inactivated by means of adding a volume of α-MEM containing serum and homogenizing the content.

The MSCs were then isolated by means of centrifugation at 500 g for 10 minutes, removing the supernatant and obtaining the cell pellet which was again resuspended in 30 ml of α-MEM and centrifuged at 500 g.

The sedimented cell pellet was resuspended in α-MEM for seeding the cells in a 250 cm² flask at 100000 cells/cm² in α-MEM supplemented with 2 mM of L-glutamine (BioWhittaker, Switzerland), 15% fetal bovine serum (FBS) (BioWhittaker, Switzerland), 100 U/ml of penicillin (Penilevel laboratories ERN, Barcelona, Spain), 0.1 mg/ml of streptomycin (laboratorios Normon, Seville, Spain) and 1 ng/ml of basic fibroblast growth factor (b-FGF; CE Peprotech, London, United Kingdom), and incubated at 37°C and 5% CO₂ in a saturated wet atmosphere.

24 hours after seeding the culture, the culture medium was replaced with α-MEM supplemented with 10% FBS and 1 ng/ml of b-FGF. The culture medium was replaced twice a week, performing washings with PBS beforehand to eliminate cells that do not adhere to the plastic. Once the cells reached 90% of confluence, they were disassociated with TryPLE Select (Life Technologies).

The cells were then centrifuged, eliminating the supernatant. The pellet was resuspended in Hank's Balanced salt solution (Sigma-Aldrich, St. Louis MO). This last step was performed twice. In the second washing, the cells were resuspended at a concentration of 7 millions/ml. Each ml of cells was sonicated in three pulses of 30 seconds with rest in ice of 15 seconds. A trypan blue assay was performed after the sonication of each vial to check for the complete absence of intact, unlysed cells.

The content of the 1 ml vial of 7 millions of lysed adipose tissue MSCs is the dose that will be frozen for subsequent infusion in the damaged joints.

The results obtained by the researchers reveal that allogeneic therapy with lysates of MSCs originating from adipose tissue or bone marrow is an effective treatment for muscle-skeletal diseases, this therapy being novel and promising in the treatment of OA, reducing the feeling of pain, improving functionality, joint movement and the quality of life of the patient without the onset of adverse effects.

Therapy with MSC lysates was evaluated in 6 dogs of the labrador retriever and German shepherd breeds (4 and 2, respectively) with a mean weight of 32.8 kg (31-40 kg) and age of 8.4 years old (6-10 years old), affected by osteoarthritis in both coxofemoral joints with a moderate degree rating based on clinical and radiological signs. The patients were evaluated before applying the treatment (baseline) and 1 and 3 months after the intra-articular infiltrations of MSC lysates. A standardized questionnaire was used at all times and the owner took the questionnaire in order to assess the degree of physical joint functionality, pain and stiffness, with values from 0 to 4 for each question. This questionnaire is a modification of the WOMAC (*Western Ontario and McMaster Universities Arthritis Index*) scale used in human medicine for evaluating the condition of patients with hip and knee osteoarthritis:

### FUNCTIONALITY STUDY QUESTIONNAIRE. BIOLOGICAL TREATMENT FOR OA

| | |
|---|---|
| PATIENT ....................................... | AGE ..................... |

### FUNCTIONAL CAPACITY

### 1. HAVE YOU OBSERVED ANY CHANGE IN YOUR PET'S ATTITUDE OR CHARACTER?:

**0= It is much happier and much more active than before treatment**
**1= It is happier and more active than before treatment**
**2= It is a bit happier and a bit more active than before treatment**
**3= It has the same attitude as before treatment**
**4= It has worse attitude than before treatment**

### 2. BEHAVIOR

**0= Happy, playful, reacts enthusiastically when called**
**1= Alert, clear response to stimuli (toys, food, etc.) or when called**
**2= Slight response when called to go for a walk, to go after a toy or to look for food**
**3= Depressed, dejected, no respond to stimuli (game, desire to go for a walk, etc.)**
**4= Bad character, even becoming aggressive when being approached**

### 3. DEGREE OF LIMPING WHILE WALKING (after walking for more than 10 minutes):.............

**0= Does not limp**
**1= Limps a little: occasional difficulty while walking**
**2= Limps quite a lot**
**3= Limps a lot: continuously walks in abnormal manner and/or raises paw up high intermittently**
**4= limps severely: keeps paw up high at the time**

### 4. RESISTANCE WHILE WALKING DURING WALK: ................

**0= Can walk long with no problems**
**1= Can walk long distances but often stops during walk**
**2= Can only go on short walks**
**3= Can only go on very short walks**
**4= Does not need to go on walks**

### 5. GOING UP OR DOWN STAIRS: ................

**0= Goes up long flights of stairs with no problems**
**1= Goes up short flights well but shows difficulty in stretched of 10 steps or more**
**2= Goes up flights of stairs with difficulty**
**3= Goes up flights of stairs with extreme difficulty (a few steps or even curbs)**
**4= Refuses to go up any stair**

### 6. CHANGES IN THE SUPPORTS IN THE STATION (rear limbs): ...............

**0= The dog bears its weight normally in the station**
**1= It bears its weight well but tries to relieves it by moving the claws on one limb to one side**
**2= Barely bears its own weight, only with the claws**
**3= Shifts the load to the other side**
**4= Does not bear its own weight on the limb**

### 7. HOW YOUR PET STANDS UP: ................

**0= The dog stands up perfectly**
**1= It stands up well but with slight difficulty**
**2= It stands up with quite a lot of difficulty**
**3= It stands up with a lot of difficulty**
**4= It does not stand up**

### 8. HOW YOUR PET LIES DOWN: ................

**0= The dog lies down perfectly**
**1= It lies down well but with slight difficulty**
**2= It lies down with quite a lot of difficulty**
**3= It lies down with a lot of difficulty**
**4= It lies down without control, letting itself fall**

### 9. LIMPING IN COLD CONDITION: ................

**0= Does not limp when it starts to walk**
**1= Limps a little at first but stops limping during the first minutes of walking**
**2= Limps quite a lot at first but stops limping in 10 minutes of walking**
**3= Limps a lot at first but stops limping after 10 minutes of walking**
**4= Limps all the time**

### 10. RESISTANCE TO RACE AND GAME: ................

**0= Can run and play with no difficulty**
**1= Runs and plays with slight difficulties**
**2= Runs or plays with quite a lot of difficulty and tires easily**
**3= Runs and plays with a lot of difficulty and tires easily**
**4= Does not run or play under any stimulus**

### 11. LIMITATIONS WITH RESPECT TO SHORT LEAPS (40-50 cm): ................

**0= Goes up the sofa or gets in-out of car with no problems**
**1= Goes up the sofa or gets in-out of car with a little difficulty**
**2= Goes up the sofa or gets in-out of car although with quite a lot of difficulty**
**3= Goes up the sofa or gets in-out of car although with a lot of difficulty**
**4= Does not go up the sofa or get in-out of car**

### SUBTOTAL OF FUNCTIONAL LIMITATION SCORE............

### JOINT MOBILITY

### 12. PAIN IN THE PASSIVE MANUAL MOBILIZATION OF JOINT: ................

**0= Absent, free of pain and crackling**
**1= Mild, mild pain occurs with manipulation**
**2= Quite a lot of pain particularly in the last degrees of bending and extension**
**3= A lot of pain and/or crackling from minimal manipulation**
**4= Manipulation impossible due to unbearable amount of pain and/or crackling**

### 13. ROM-LIMITATION OF BENDING MOVEMENT, THE DEGREE OF BENDING OF THE KNEE IS:

**0= Completely bended at 50-60°**
**1= Slight limitation < 80°**
**2= Severe limitation > 80°**

### 14. ROM-LIMITATION OF EXTENSION MOVEMENT, THE GRADO OF EXTENSION OF THE KNEE IS: ................

**0= Complete extension at 160-170°**
**1= Slight limitation 150-160°**
**2= Severe limitation < 150°**

### SUBTOTAL OF JOINT MOBILITY SCORE

### MUSCLE ATROPHY

### 15. MUSCLE ATROPHY:

**0= No signs of muscle atrophy**
**1= Mild atrophy**
**2= Moderate atrophy**
**3= Severe atrophy**

Likewise, the visual analogue scale (VAS) done by the owner and the veterinarian was included for the diagnosis of the intensity of pain experienced by the patient:

### SUBJECTIVE PAIN ASSESSMENT (VAS)

### 0 corresponding to NO PAIN and 100 corresponding to EXTREME PAIN

Draw a vertical mark on the line corresponding to the value of PAIN I think MY PET EXPERIENCES at this moment

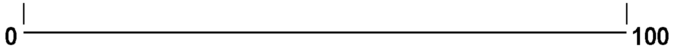 0 100

| | | |
|---|---|---|
| **Would the owner recommend stem cell treatment?** | **YES** | **NO** |
| **Did the pet required taking any analgesic at this time?** | **YES** | **NO** |
| **OBSERVATIONS (anything to be specified)** | | |

Furthermore, the range of joint mobility was evaluated by means of goniometry, measuring the angles of bending and extension of each hip. The degree of muscle atrophy was also evaluated, monitoring the muscular perimeter measured in the muscle for each pelvic limb. The use of the dynamometric platform allowed studying kinetics parameters of the patient by means of recoding and analyzing the reactive force the rear limbs of the patient exert on the ground while performing the movement: Peak vertical force (PVF), vertical impulse (VI), bearing time. Finally, after follow-ups at 1 and 3 months, the owners answered a questionnaire that included six questions, with 5 graduated levels of score from very well to very poor, about the satisfaction based on the patients' response to treatment, and the detection of possible therapy-related adverse effects:

### ASSESSMENT OF OWNER SATISFACTION

**1. In your opinion, how has the limping your pet experiences progressed?**

| | | | | |
|---|---|---|---|---|
| **Very well** | **Well** | **Regular** | **Poor** | **Very poor** |

**2. Treatment tolerance**

| | | | | |
|---|---|---|---|---|
| **Very well** | **well** | **Regular** | **Poor** | **Very poor** |

**3. In your opinion, how has your pet responded to the treatment this time?**

| | | | | |
|---|---|---|---|---|
| **Very well** | **Well** | **Regular** | **Poor** | **Very poor** |

**4. In your opinion, how has the degree of limping your pet experiences progressed?**

| | | | | |
|---|---|---|---|---|
| **Very well** | **Well** | **Little** | **Poor** | **Very poor** |

**5. In your opinion, is stem cell treatment effective up until now?**

| | | | | |
|---|---|---|---|---|
| **Very well** | **Well** | **Regular** | **Poor** | **Very poor** |

**6. In your opinion, how limited is your pet's quality of life?**

| | | | | |
|---|---|---|---|---|
| **Not at all** | **Slightly limited** | **Fairly limited** | **Somewhat limited** | **Very limited** |

The results of the therapy were statistically quantified and analyzed, revealing an excellent effect. The weight of the animals did not show significant changes either after 1 month or after 3 months with respect to the baseline. The results of the questionnaires for the functional capacity showed improvement after 1 month with respect to the baseline condition, said results were maintained after 3 months. This questionnaire reveals that the functionality of the patients improves significantly after 1 month with a median of 0 (0-1), they also experience change in attitude and character, being much happier and much more active than before treatment, play and react enthusiastically when called, stop limping, can walk long distances with no problems, go up and down of long flights of stairs with no difficulty, stand up and lie down perfectly, run and play with no difficulty and with no limitation in short leaps, with respect to the baseline assessment with a median of 2 (2-3), in which the patients were less active and happy, have slight response when called or asked to play, with obvious limping, can only go on short walks, go up flights of stairs with quite a lot of difficulty, stand up and lie down with quite a lot of difficulty, run and play with difficulty and tire easily, have quite a lot of difficulty when taking short leaps.

After 1 month (median of 1, 1-2), the range of joint mobility of the patients improved with respect to the baseline condition (median 2, 2-3) as they express less pain to passive mobilization of the coxofemoral joints, this improvement is maintained after 3 months (median of 1, 0-1). The increase in joint mobility is more obvious in the evaluation of the degree of extension, particularly after 3 months (right extension of 147.5°, left extension of 160°) with respect to the baseline (right extension of 130°, left extension of 125°) (Figure 1). The measurement of the muscular perimeter of the muscle for each pelvic limb did not demonstrate changes when comparing the different times of study. The VAS monitored a decrease in the average assessment of pain in the patient after 1 month (2.1) and 3 months (1.2) with respect to the baseline (6.8) (see Figure 2). The analysis of the movement with the dynamometric platform is also shown in the graphs of annex 4. A significant improvement in the PFV (peak vertical force) was detected (Nw/kg, right: baseline 6.3, 1 month 6.3, 3 months 6.4; left: baseline 6.8, 1 month 6.8, 3 months 6.4) (Figure 3) and the IV (vertical impulse) (Nw/sg, right: baseline 0.89, 1 month 0.92, 3 months 0.95; left: baseline 0.87, 1 month 0.91, 3 months 0.91) relating the reaction forces on the floor of both pelvic limbs after therapy with lysate of MSC (Figure 3). In all the treated patients, the therapy was satisfactory with clear signs of improvement and no adverse effect whatsoever was detected after three months of study. Both after 1 month and after 3 months, the owners assessed the tolerance of their animal to MSC treatment as very good, the treatment response as very good, the progress of the limping as very good, the efficacy of the MSC therapy as very good, and clear improvement their pet's quality of life in relation to the condition of the patients before treatment with lysate of MSC.

Based on all the studied parameters, it can be concluded that the lysates of MSC improve osteoarthritis injury, proposing them as an effective treatment for more than three months. In view of the beneficial effects found, this treatment with lysates of MSC is thought to have the capacity for improving not only osteoarthritis injuries but also other of type tendon and cartilage injuries observed both in dogs, horses and humans.

It has been discovered that, like intact MSC, the cell lysate thereof triggers a beneficial effect, even accelerates regeneration process and prevents a possible detrimental side effect derived from mutations or changed occurring in the introduced cells.

This fact represents a completely novel therapy concept which provides the following advantages:
1. The beneficial effect of the MSCs develops more rapidly since a concentrate of the factors released by the living or intact MSCs is infused in the site of the injury.
2. Using lysed mesenchymal stem cells eliminates the possibility of there being possible adverse effects derived from the transformation of the stem cells infused in tumor cells or undesired cell types.

Since it is an allogeneic infusion, the administration of a concentrate of this cell lysate is made immediately possible (once the injury is assessed). The pharmaceutical composition only has to be thawed locally. This eliminates other processes performed by the competition such as the need to extract large amounts of blood, bone marrow or adipose tissue from the patient. Furthermore, It accelerates the availability of treatment and prevents major handling of the animals without the need of hospitalization or surgery.

## Claims

1. An intracellular content of at least one isolated mesenchymal stem cell for use in the prevention, relief or treatment of osteoarthrosis, wherein the isolated mesenchymal stem cell is obtained from a different individual from which the intracellular content will be administered and wherein said intracellular content of at least one isolated mesenchymal stem cell is infused in the site of injury.

2. The intracellular content of at least one isolated mesenchymal stem cell for use according to claim 1, wherein the isolated mesenchymal stem cell is obtained from a mammal, preferably a dog, horse or human.

3. A composition comprising the intracellular content of any of claims 1 or 2, for use according to any of claims 1 or 2.

4. The composition for use according to claim 3, wherein the composition further comprises a further active ingredient or therapeutic agent.

5. The composition for use according to the preceding claim, wherein the further therapeutic agent is selected from an analgesic agent or an anti-infective agent.

## Patentansprüche

1. Intrazellulärer Inhalt mindestens einer isolierten mesenchymalen Stammzelle für dessen Verwendung bei der Vorbeugung, Linderung oder Behandlung von Osteoarthrose, wobei die isolierte mesenchymale Stammzelle aus einem unterschiedlichen Individuum erhalten wird, als welchem der intrazelluläre Inhalt verabreicht werden wird und wobei der genannte intrazellulärer Inhalt mindestens einer isolierten mesenchymalen Stammzelle der Verletzungsstelle infundiert wird.

2. Intrazellulärer Inhalt mindestens einer isolierten mesenchymalen Stammzelle für dessen Verwendung nach Anspruch 1, wobei die isolierte mesenchymale Stammzelle aus einem Säugetier, vorzugsweise einem Hund, Pferd oder Menschen erhalten wird.

3. Zusammensetzung umfassend den intrazellulären Inhalt nach einem der Ansprüche 1 oder 2, für dessen Verwendung nach einem der Ansprüche 1 oder 2.

4. Zusammensetzung für dessen Verwendung nach Anspruch 3, wobei die Zusammensetzung zusätzlich einen weiteren Wirkstoff oder ein weiteres Therapeutikum umfasst.

5. Zusammensetzung für dessen Verwendung nach dem vorhergehenden Anspruch, wobei das weitere Therapeutikum aus einem schmerzlindernden Mittel oder einem antiinfektiösen Mittel ausgewählt wird.

## Revendications

1. Contenu intracellulaire d'au moins une cellule souche mésenchymateuse isolée pour son utilisation dans la prévention, le soulagement ou le traitement de l'ostéoarthrose, dans lequel la cellule souche mésenchymateuse isolée est obtenue à partir d'un individu différent de celui auquel le contenu intracellulaire sera administré et dans lequel ledit contenu intracellulaire d'au moins une cellule souche mésenchymateuse isolée est infusé dans le site de lésion.

2. Contenu intracellulaire d'au moins une cellule souche mésenchymateuse isolée pour son utilisation selon la revendication 1, dans lequel la cellule souche mésenchymateuse isolée est obtenue à partir d'un mammifère, préférablement un chien, un cheval ou un humain.

3. Composition qui comprend le contenu intracellulaire selon l'une quelconque des revendications 1 ou 2, pour son utilisation selon l'une quelconque des revendications 1 ou 2.

4. Composition pour son utilisation selon la revendication 3, dans laquelle la composition comprend en outre un principe actif ou un agent thérapeutique supplémentaire.

5. Composition pour son utilisation selon la revendication précédente, dans laquelle l'agent thérapeutique supplémentaire est sélectionné parmi un agent analgésique ou un agent anti-infectieux.
